(19) Europäisches Patentamt
European Patent Office
Office européen des brevets

(11) Numéro de publication: 0 200 638
B1

(12) # FASCICULE DE BREVET EUROPÉEN

(45) Date de publication du fascicule du brevet: 19.04.89

(51) Int. Cl.⁴: C 07 C 103/737

(21) Numéro de dépôt: 86400673.5

(22) Date de dépôt: 22.04.86

(54) **Procédé de préparation du chlorhydrate de phényl-1 diéthyl amino carbonyl-1 aminométhyl-2 cyclopropane (Z).**

(30) Priorité: 25.04.85 FR 8506335

(43) Date de publication de la demande: 05.11.86 Bulletin 86/45

(45) Mention de la délivrance du brevet: 19.04.89 Bulletin 89/16

(84) Etats contractants désignés: AT BE CH DE FR GB IT LI LU NL SE

(56) Documents cité:
EP-A-0 068 999

BOLL. CHIM. FARM., vol. 117, no. 6, 1978, pages 331-342; S. CADASIO et al.: "Acide phenyl-1-hydroxymethyl-2-cyclopropane carboxylique et derivés"
CHEMISCHE BERICHTE, vol. 83, no. 3, 1950, pages 244-247, Weinheim, DE; W. GRASSMANN et al.: "Die Synthese einiger Peptide nach dem Phthalyl-Verfahren"

(73) Titulaire: PIERRE FABRE MEDICAMENT, 125, Rue de la Faisanderie, F-75116 Paris (FR)

(72) Inventeur: Bonnaud, Bernard, 2 rue Georges Bizet, F-81100 Castres (FR)
Inventeur: Mouzin, Gilbert, 21 rue Saint Foy, F-81100 Castres (FR)
Inventeur: Cousse, Henri, La Foun de los Nobios Chemin de Lastinos, F-81100 Castres (FR)
Inventeur: Patoiseau, Jean- François, 7, rue Jules Ferry, F-81100 Castres (FR)

(74) Mandataire: Ahner, Francis, CABINET REGIMBEAU 26, avenue Kléber, F-75116 Paris (FR)

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).



LIBER, STOCKHOLM 1989

## Description

La présente invention réalisée au Centre de Recherches P.F. MEDICAMENT concerne un nouveau procédé industriel de préparation du MIDALCIPRAN (dénomination commune internationale du chlorhydrate de phényl-1, diéthyl amino carbonyl-1 aminométhyl-2- cyclopropane "Z") répondant à la formule I:

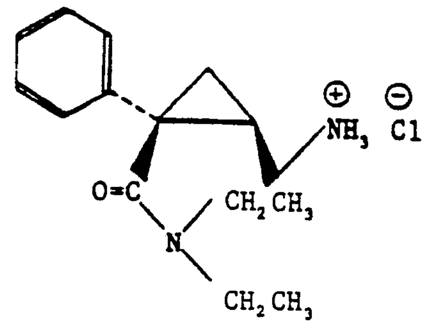

I

Le MIDALCIPRAN est actuellement développé en clinique comme antidépresseur.

Dans la technique antérieure, illustrée par FR-A-2 508 035, ce composé était préparé selon un procédé en 5 étapes, à partir du phényl-1 oxo-2 oxa-3 bicyclo (3 : 1 : 0) hexane, ce dernier dérivé étant décrit par FR-A-2 302 994.

Conformément à cette technique antérieure, l'étape limitante du procédé de préparation se situe au stade de la réaction du chlorure de chlorhydrate de phényl-1 aminométhyl-2 cyclopropane sur la diéthylamine. Au cours de cette réaction, il se produit en effet une réaction secondaire, à savoir la cyclisation intramoléculaire qui conduit à la formation d'un lactame: le phényl-1 oxo-2 aza-3 bicyclo (3 : 1 : 0) hexane.

**Schema de la réaction secondaire parasite**

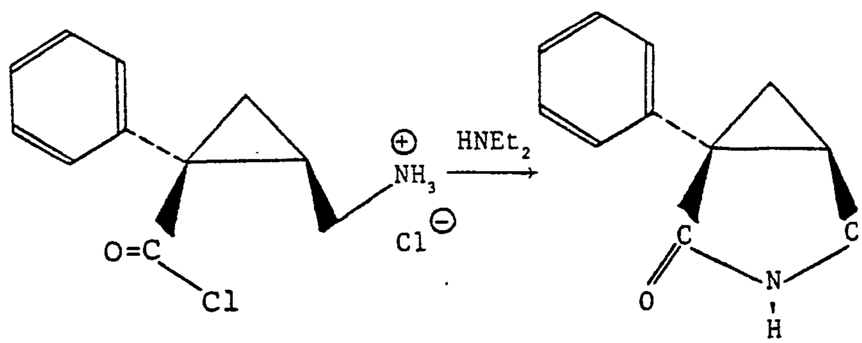


L'élimination du lactame nécessite une purification supplémentaire qui abaisse le rendement en MIDALCIPRAN.

L'objet de la présente invention concerne un nouveau procédé industriel de préparation de MIDALCIPRAN en 3 stades de synthèse. Ce procédé est caractérisé par le fait que, dès la première étape, il y a création de la fonction amine sous une forme protégée permettant ainsi l'activation de la fonction carboxylique en vue de la condensation sur la diéthylamine sans formation de produit secondaire.

Le procédé de l'invention peut être schématisé comme suit:

**Schema réactionnel**

**1ère étape**

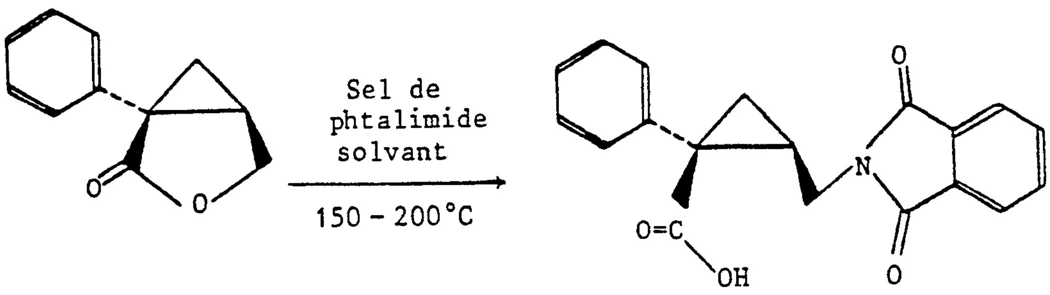


(II)

(III)

**2ème étape**

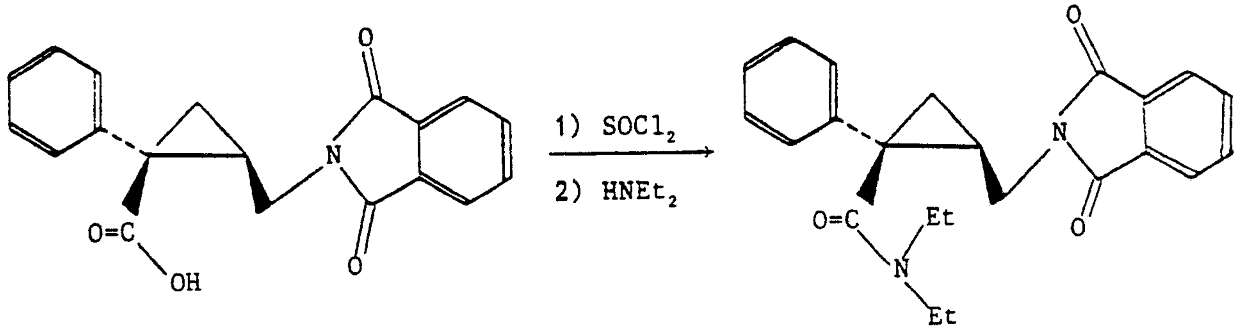


(III)

(IV)

**3ème étape**

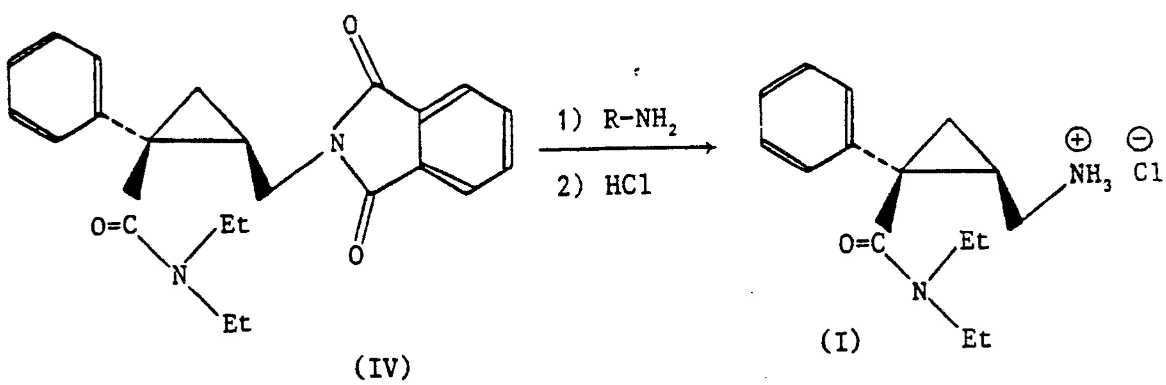


(I)

(IV)

La première étape du procédé consiste en la condensation de la lactone de formule II avec un sel de phtalimide, en particulier le phtalimide de potassium, au sein d'un solvant organique de préférence choisi parmi le diméthylformamide, le diméthylacétamide et la méthylpyrrolidone. La température réactionnelle est de préférence comprise entre 150° et 200° C et le temps de réaction varie de 5 à 15 heures.

Au cours de la seconde étape réactionnelle, l'acide de formule III est traité par un chlorure d'acide, en particulier le chlorure de thionyle, de préférence utilisé en excès stoechiométrique. La réaction est avantageusement conduite par chauffage au reflux, pendant une période comprise entre 0,5 et 2 heures. Après élimination du chlorure de thionyle, le chlorure d'acide formé est condensé sur la diéthylamine au sein d'un solvant organique, de préférence choisi parmi le chlorure de méthylène, le chloroforme, le tétrahydrofuranne et le dioxanne. La température de cette amidification est avantageusement comprise entre 5 et 30° C.

Au cours de la troisième étape réactionnelle, la fonction amine primaire se trouve libérée par traitement de l'amide de formule IV avec une alcoylamine ou une hydroxyalcoylamine de faible masse molaire, en particulier l'éthanolamine, utilisée seule ou en présence d'un solvant tel que l'eau ou un alcool de bas poids moléculaire (méthanol, éthanol, propanol, isopropanol). Cette réaction est conduite à une température comprise entre la température ambiante et le point d'ébullition du milieu réactionnel.

Après extraction du milieu réactionnel par un solvant tel que le chlorure de méthylène, le chloroforme ou l'acétate d'éthyle, l'huile résiduelle obtenue après élimination du solvant est chlorhydratée par exemple à l'aide d'une solution éthan olique d'acide chlorhydrique et le composé de formule I est cristallisé par addition d'éther, d'éther isopropylique ou de méthylal.

**Mode operatoire**

**1ère étape**

Acide phényl-1 phtalimido méthyl-2 cyclopropane carboxylique (Z) (composé III)

La suspension de 52,56 g (0,3 mole) de phényl-1 oxo-2 oxa-3 bicyclo (3 : 1 : 0) hexane (composé II) et 61 g (0,33 mole de phtalimide de potassium dans 270 $cm^3$ de diméthylformamide est maintenue sous agitation à 150° C pendant 12 heures.

La solution obtenue après retour à température ambiante est versée dans 1000 $cm^3$ d'eau. Après extraction par l'acétate d'éthyle, la phase aqueuse est acidifié par un excès d'acide acétique puis glacée. L'acide qui cristallise est essoré, lavé à l'eau et recristallisé dans l'éthanol pour donner 62,6 g de composé III (rendement: 65 %).

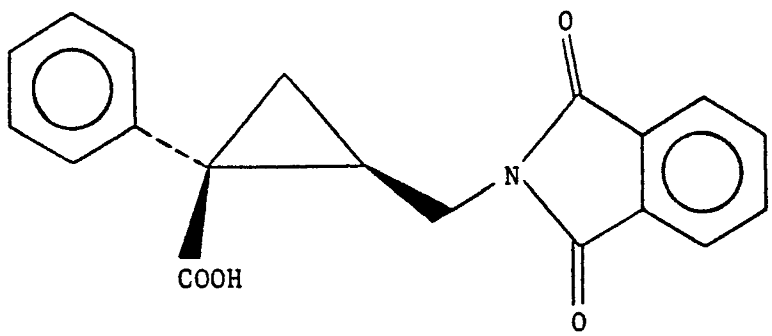

Point de fusion: 186°
Formule: $C_{19}H_{15}NO_4$: 321,22
CCM (silice - GF 254 Merck)
Rf: 0,6 (chloroforme 85 - méthanol
IR: (KBr) ν C = O: 1775, 1710 et 1650 $cm^{-1}$
$^1H$ RMN ($CDCl_3$) δ ppm (TMS): 1,1 à 2 (m, 3H, cyclopropaniques); 3,9 (d, 2H), $CH_2N$); 7,15 (s, 5H, aromatiques); 7,75 (s, 4H, aromatiques).

**2ème étape**

Phényl-1 diéthylamino carbonyl-1 phtalimido méthyl-2 cyclopropane (Z) (composé IV)

Dans 30 $cm^3$ de chlorure de thionyle, sous agitation à température ambiante, sont ajouté par fraction 16,2 g (0,05 mole) d'acide phényl-1-phtalimido méthyl-2 cyclopropane carboxylique (composé III). On obtient une dissolution après 2 heures à température ambiante.

La solution est alors maintenue au reflux pendant 2 heures. Après élimination du chlorure de thionyle en excès, le chlorure d'acide brut en solution dans 50 $cm^3$ de chlorure de méthylène est introduit goutte à goutte dans la solution de 10,3 $cm^3$ (0,1 mole) de diéthylamine et 150 $cm^3$ de chlorure de méthylène sous agitation sur bain de glace. Après une nuit sous agitation à température ambiante, la solution obtenue est lavée à l'eau, séché sur $Na_2SO_4$, filtrée et concentrée sous pression réduite. Par addition d'éther isopropylique, on obtient 15,4 g de composé IV (rendement: 82 %).

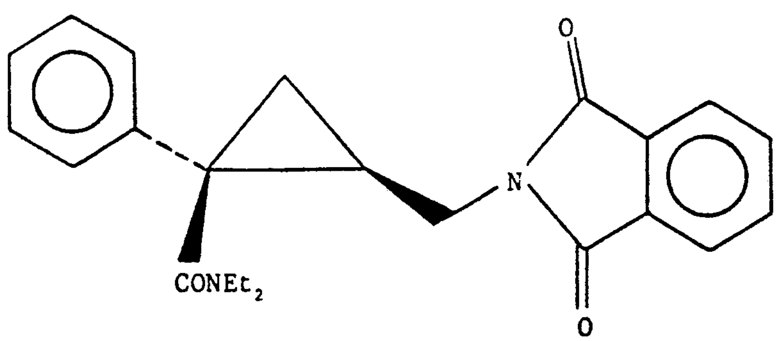

Point de fusion 131°
Formule: $C_{23}H_{24}H_2O_3$: 376,46
CCM (silice GF 254 Merck);

Rf: 0,66 (chloroforme 95 - méthanol 5)
IR (KBr) ν C = O 1630, 1705 et 1770 $cm^{-1}$
$^1H$ RMN ($CDCl_3$) δ ppm (TMS): 0,65 (t, 3H, $CH_3$); 1,15 (t, 3H, $CH_3$); 1,25 (m, 1H, cyclopropaniques); 1,5 - 2,3 (m, 2H, cyclopropaniques); 3 - 3,7 (m, 5H, $CH_2N$, cyclo-CH-N); 4,15 (dd, 1H, cyclo-CH-N); 7,1 (s, 5H, aromatiques); 7,6 (m, 4H, aromatiques).

**3ème étape**

Phényl-1 diéthylamino carbonyl-1 aminométhyl-2 cyclopropane (Z) chlorhydrate (composé I). MIDALCIPRAN

a) La suspension de 18,82 g (0,05 mole) de phényl-1 diéthylamino carbonyl-1 phtalimido méthyl-2 cyclopropane (composé IV) dans 95 $cm^3$ de solution aqueuse de méthylamine à 40 % est maintenué sous agitation à température ambiante pendant 5 heures.

Dissolution puis cristallisation du N,N-diméthyl phtalamide. La suspension est extraite 3 fois par l'acétate d'éthyle. La phase organique est lavée à l'eau, séchée sur $Na_2SO_4$, filtrée et le solvant est éliminé sous pression réduite. Après addition d'une solution éthanolique d'acide chlorhydrique puis d'éther, on obtient 10 g de cristaux du composé I (rendement: 71 %).

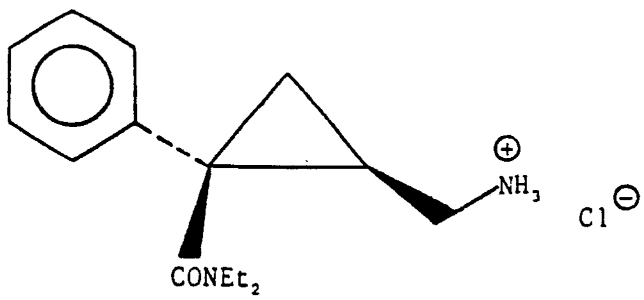

Point de fusion: 180°
Formule: $C_{15}H_{23}ClN_2O$: 282,82
CCM (silice GF 245 Merck)
Rf: 0,43 (chloroforme 84 - méthanol 14 - NH OH)
IR (KBr): ν C = O 1610 $cm^{-1}$
$^1H$ RMN ($D_2O$) δ ppm (T.S.P.) 0,8 (t, 3H, $CH_3$); 1,15 (t, 3H, $CH_3$); 1,5 - 2,1 (m, 3H, cyclopropanique); 3 - 3,7 (m, $CH_2N$); 7,3 (s, aromatiques).

b) La suspension de 60 g (0,159 mole) de phényl-7 diéthyl-amino carbonyl-1 phtalimido méthyl-2 cyclopropane dans 60 $cm^3$ d'éthanolamine est maintenue pendant 1 heure à 90°C sous agitation.

Après addition de 300 ml d'eau glacée, on extrait 3 fois par l'acétate d'éthyle.

La phase organique est lavée à l'eau, séchée sur $Na_2SO_4$, filtrée et le solvant éliminé sous pression réduite.

Après addition d'une solution éthanolique d'acide chlorhydrique 3N puis d'éther, on obtient 39,7g de cristaux du composé I (Rendement 88 %).

Point de fusion: 178 - 180°C.

**Revendications**

1. Procédé pour la préparation industrielle du chlorhydrate de phényl-1 diéthylamino carbonyl-1 aminométhyl-2 cyclopropane (Z) de formule I:

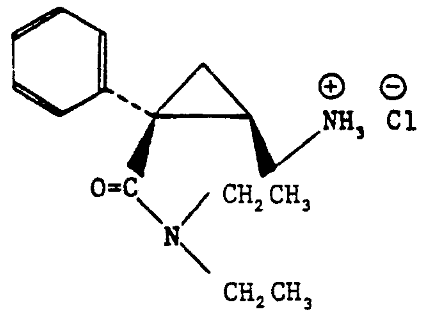

I

caractérisé par la mise en oeuvre des étapes réactionnelles successives suivantes:
i) le phényl-1 oxo-2 oxa-3 bicyclo (3 : 1 : 0) hexane de formule II:

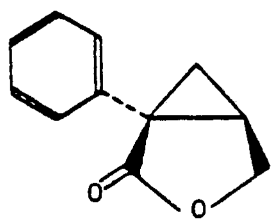

II

est ouvert par traitement à l'aide d'un sel de phtalimide au sein d'un solvant organique:
ii) l'acide phényl-1 phtalimido méthyl-2 cyclopropane carboxylique (Z) de formule III ainsi obtenu

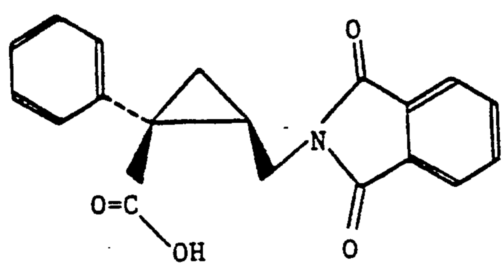

III

est traité par un chlorure d'acide, puis le chlorure d'acide ainsi formé est amidifié par condensation avec la diéthylamine au sein d'un solvant organique; et
iii) le phényl-1 diéthylamino carbonyl-1 phtalimido méthyl-2 cyclopropane (Z) de formule IV ainsi obtenu:

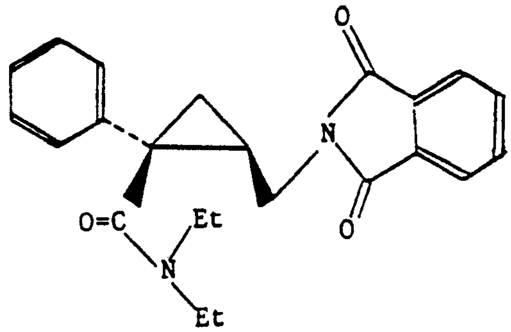

IV

est ensuite traité par une alcoylamine ou une hydroxyalcoylamine primaire seule ou en présence d'un solvant pour obtenir, après chlorhydratation, le composé de formule I précitée.

2. Procédé selon la revendication 1, caractérisé en ce que le sel de phtalimide utilisé est le phtalimide de potassium.

3. Procédé selon l'une des revendications 1 et 2, caractérisé en ce que le traitement de la lactone de formule II par le sel de phtalimide est réalisé au sein d'un solvant organique choisi parmi le diméthylformamide, le diméthylacétamide et la méthylpyrrolidone.

4. Procédé selon l'une des revendications 1 à 3, caractérisé en ce que la première étape de condensation du sel de phtalimide avec la lactone de formule II est effectuée à une température réactionnelle située dans la zone des points d'ébullition du solvant utilisé au cours de cette étape, de préférence comprise entre 150° C et 200° C.

5. Procédé selon l'une des revendications 1 à 4, caractérisé en ce que le chlorure d'acide utilisé au cours de la seconde étape réactionnelle est le chlorure de thionyle.

6. Procédé selon l'une des revendications 1 à 5, caractérisé en ce que le traitement du composé de formule III par le chlorure d'acide est réalisé par chauffage au reflux.

7. Procédé selon l'une des revendications 1 à 6, caractérisé en ce que l'amidification de la seconde étape réactionnelle est effectuée au sein d'un solvant organique choisi parmi le chlorure de méthylène, le chloroforme, le tétrahydrofuranne et le dioxanne.

8. Procédé selon l'une des revendications 1 à 7, caractérisé en ce que la température réactionnelle de l'amidification de la seconde étape est comprise entre 5° C et 30° C.

9. Procédé selon l'une des revendications 1 à 8, caractérisé en ce que l'amine primaire utilisée au cours de la troisième étape réactionnelle est l'éthanolamine.

10. Procédé selon l'une des revendications 1 à 9, caractérisé en ce que la chlorhydratation finale est obtenue à l'aide d'une solution éthanolique d'acide chlorhydrique.

**Claims**

1. Process for the industrial preparation of (Z)-1-phenyl-1-diethylaminocarbonyl-2-aminomethylcyclopropane of formula I:

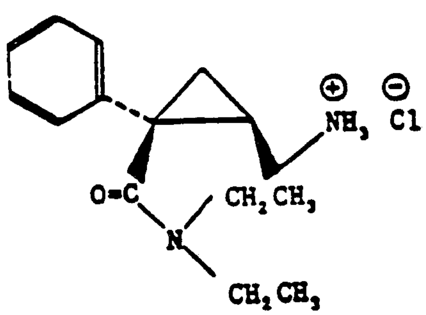

I

characterized by the use of the following successive reaction steps:

i) 1-phenyl-2-oxo-3-oxa(3 : 1 : 0)bicyclohexane of formula II:

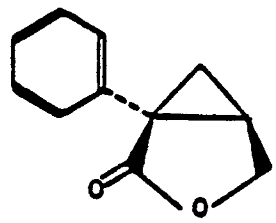

II

is opened by treatment with a phthalimide salt in an organic solvent;

ii) (Z)-1-phenyl-2-phthalimidomethylcyclopropane-carboxylic acid of formula III thus obtained

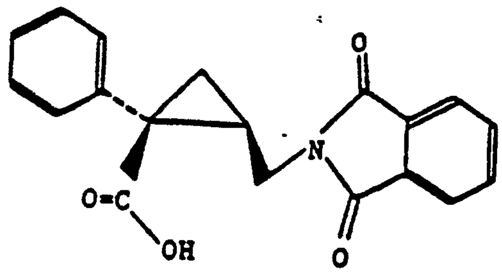

III

is treated with an acid chloride, and then the acid chloride thus formed is amidified by condensation with diethylamine in an organic solvent; and

iii) (Z)-1-phenyl-1-diethylaminocarbonyl-2-phthalimidomethylcyclopropane of formula IV thus obtained:

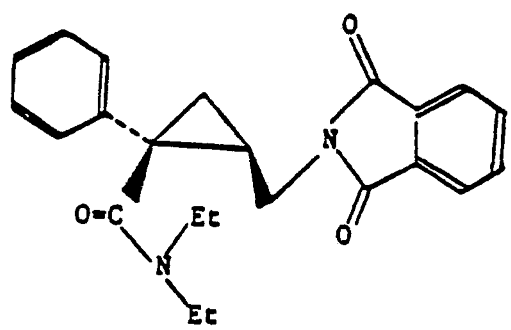

IV

is then treated with an alkylamine or a primary hydroxyalkylamine by itself or in the presence of a solvent to obtain, after hydrochlorination, the compound of abovementioned formula I.

2. Process according to Claim 1, characterized in that the phthalimide salt employed is potassium phthalimide.

3. Process according to either of Claims 1 and 2, characterized in that the treatment of the lactone of formula II with the phthalimide-salt is performed in an organic solvent chosen from dimethylformamide, dimethylacetamide and methylpyrrolidone.

4. Process according to one of Claims 1 to 3, characterized in that the first step of condensation of the phthalimide salt with the lactone of formula II is performed at a reaction temperature situated in the range of the boiling points of the solvent employed during this step, preferably between 150° C and 200° C.

5. Process according to one of Claims 1 to 4, characterized in that the acid chloride employed during the second reaction step is thionyl chloride.

6. Process according to one of Claims 1 to 5, characterized in that the treatment of the compound of formula III with the acid chloride is performed by heating under reflux.

7. Process according to one of Claims 1 to 6, characterized in that the amidification of the second reaction step is performed in an organic solvent chosen from methylene chloride, chloroform, tetrahydrofuran and dioxane.

8. Process according to one of Claims 1 to 7, characterized in that the reaction temperature of the amidification in the second step is between 5° C and 30° C.

9. Process according to one of Claims 1 to 8, characterized in that the primary amine employed during the third reaction step is ethanolamine.

10. Process according to one of Claims 1 to 9, characterized in that the final hydrochlorination is effected

with the aid of an ethanolic solution of hydrochloric acid.

## Patentansprüche

1. Verfahren zur industriellen Herstellung des Hydrochlorides von Phenyl-1-diethylaminocarbonyl-1-aminomethyl-2-cyclopropan (Z) der Formel I:

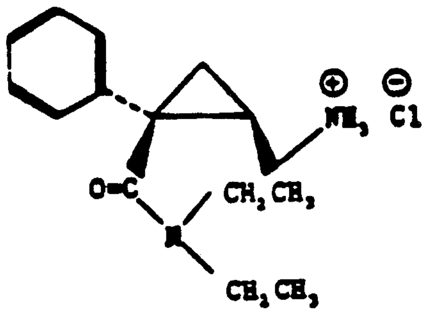

I

dadurch gekennzeichnet, daß man nacheinander die folgenden Reaktionsstufen durchführt:

i) Phenyl-1-oxo-2-oxa-3-bicyclo(3 : 1 : 0)hexan der Formel II:

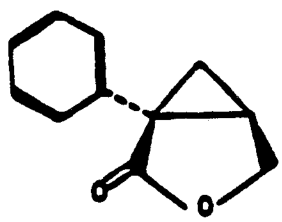

II

wird durch Umsetzung mit einem Salz des Phthalimides in einem organischen Lösungsmittel geöffnet:

ii) die Phenyl-1-phthalimidomethyl-2-cyclopropancarbonsäure (Z) der Formel III, die auf diese Weise erhalten wird,

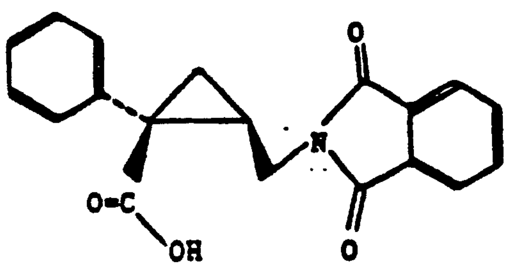

III

wird mit einem Säurechlorid behandelt, worauf das auf diese Weise erhaltene Säurechlorid durch Kondensation mit Diethylamin in einem organischen Lösungsmittel amidiert wird; und

iii) das auf diese Weise gewonnene Phenyl-1-diethylaminocarbonyl-1-phthalimidomethyl-2-cyclopropan (Z) der Formel IV

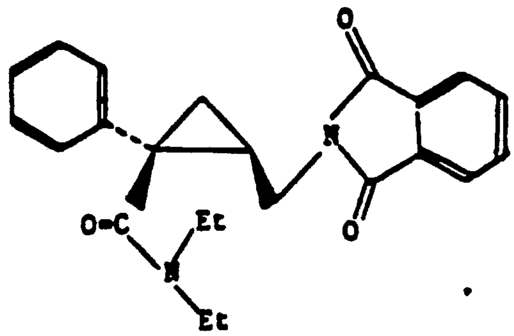

IV

wird daraufhin mit einem Alkylamin oder einem primären Hydroxyalkylamin allein oder in Gegenwart eines Lösungsmittels umgesetzt, um nach Hydrochlorierung die Verbindung der angegebenen Formel I zu gewinnen.

2. Verfahren nach Anspruch 1, dadurch gekennzeichnet, daß man als Phthalimidsalz das Kaliumsalz des Phthalimides verwendet.

3. Verfahren nach einem der Ansprüche 1 und 2, dadurch gekennzeichnet, daß man die Umsetzung des Lactons der Formel II mit dem Salz des Phthalimides in einem organischen Lösungsmittel durchführt, das ausgewählt ist aus Dimethylformamid, Dimethylacetamid und Methylpyrrolidon.

4. Verfahren nach einem der Ansprüche 1 bis 3, dadurch gekennzeichnet, daß man die erste Stufe der Kondensation des Phthalimidsalzes mit dem Lacton der Formel II bei einer Reaktionstemperatur durchführt, die im Bereich der Siedepunkte des verwendeten Lösungsmittels im Verlauf dieser Stufe liegt, vorzugsweise zwischen 150 und 200° C.

5. Verfahren nach einem der Ansprüche 1 bis 4, dadurch gekennzeichnet, daß das in der zweiten Reaktionsstufe verwendete Säurechlorid aus Thionylchlorid besteht.

6. Verfahren nach einem der Ansprüche 1 bis 5, dadurch gekennzeichnet, daß man die Umsetzung der Verbindung der Formel III mit dem Säurechlorid durch Erhitzen auf Rückflußtemperatur bewirkt.

7. Verfahren nach einem der Ansprüche 1 bis 6, dadurch gekennzeichnet, daß man die Amidierung in der zweiten Reaktionsstufe in einem organischen Lösungsmittel, ausgewählt aus Methylenchlorid, Chloroform, Tetrahydrofuran und Dioxan durchführt.

8. Verfahren nach einem der Ansprüche 1 bis 7, dadurch gekennzeichnet, daß die Reaktionstemperatur bei der Amidierung in der zweiten Stufe zwischen 5 °C und 30 °C liegt.

9. Verfahren nach einem der Ansprüche 1 bis 8, dadurch gekennzeichnet, daß das primäre Amin, das in der dritten Reaktionsstufe verwendet wird, Ethanolamin ist.

10. Verfahren nach einem der Ansprüche 1 bis 9, dadurch gekennzeichnet, daß die abschließende Hydrochlorierung mit Hilfe einer ethanolischen Lösung der Chlorwasserstoffsäure bewirkt wird.